(19) Europäisches Patentamt
European Patent Office
Office européen des brevets

(11) Publication number: 0 079 489
A1

(12) # EUROPEAN PATENT APPLICATION

(21) Application number: 82109831.6

(22) Date of filing: 25.10.82

(51) Int. Cl.[3]: G 01 N 33/78

(30) Priority: 04.11.81 US 318028

(43) Date of publication of application: 25.05.83 Bulletin 83/21

(84) Designated Contracting States: DE FR GB IT

(71) Applicant: MILES LABORATORIES INC.
1127 Myrtle Street
Elkhart Indiana 46514(US)

(72) Inventor: Albarella, James Paul
25845 Meadow Oak Lane
Elkhart, IN 46514(US)

(72) Inventor: Carrico, Robert Joseph
54256 Silver Street
Elkhart, IN 46514(US)

(74) Representative: Senftl, Hannes, Dr. et al,
c/o Bayer AG Zentralbereich Patente Marken und Lizenzen
D-5090 Leverkusen 1, Bayerwerk(DE)

(54) **Methotrexate-labeled iodothyronine conjugates and method, reagent means and test device for the determination of iodothyronine in or iodothyronine uptake of a liquid sample.**

(57) Methotrexate-labeled iodothyronine conjugates useful as labeled conjugates in specific binding assays (e.g., homogeneous immunoassays) for determining iodothyronines, particularly thyroxine and triiodothyronine, or for determining iodothyronine uptake in liquid samples. The methotrexate label is monitored preferably by measuring its inhibitory effect on the enzyme dihydrofolate reductase. Also provided are assay methods, reagent means, test kits and test devices for determining an iodothyronine, comprising the methotrexate-labeled conjugate, a binding protein (e.g., antibody) for the iodothyronine, dihydrofolate reductase, and an indicator of dihydrofolate reductase activity, and for determining iodothyronine uptake, comprising the labeled conjugate, binding protein, dihydrofolate reductase, and enzyme indicator.

EP 0 079 489 A1

Croydon Printing Company Ltd.

# METHOTREXATE-LABELED IODOTHYRONINE CONJUGATES AND METHOD, REAGENT MEANS AND TEST DEVICE FOR THE DETERMINATION OF IODOTHYRONINE IN OR IODOTHYRONINE UPTAKE OF A LIQUID SAMPLE

## BACKGROUND OF THE INVENTION

### *1. FIELD OF THE INVENTION*

This invention relates to methotrexate-labeled iodothyronine conjugates, and assay methods, reagent means, test kits, and test devices comprising such conjugates. The conjugates of the present invention are useful as labeled conjugates in specific binding assays, particularly homogeneous immunoassays, for determining iodothyronines in or iodothyronine uptake of a liquid sample, usually a biological fluid. In particular, the invention relates to methotrexate-labeled thyroxine conjugates useful in homogeneous enzyme inhibitor-labeled immunoassays for determining thyroxine in serum or plasma samples.

The iodothyronines have the following general formula:

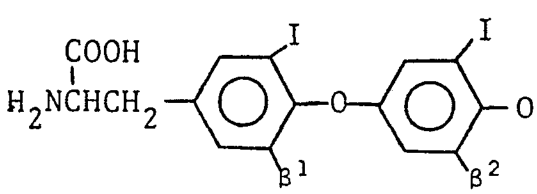

wherein $\beta^1$ and $\beta^2$ are, independently, hydrogen or

iodine. The iodothyronines of clinical interest are listed in the following table:

| Iodothyronine | $\beta^1$ | $\beta^2$ |
|---|---|---|
| 3,5,3'5'-tetraiodothyronine (thyroxine; T-4) | iodine | iodine |
| 3,5,3'-triiodothyronine (liothyronine; T-3) | iodine | hydrogen |
| 3,5',5'-triiodothyronine ("reverse" T-3) | hydrogen | iodine |
| 3,3'-diiodothyronine | hydrogen | hydrogen |

The quantitative determination of the concentration of the various iodothyronines, particularly the hormones T-3 and T-4, in serum and of the degree of saturation of the iodothyronine binding sites on the carrier protein thyroid binding globulin (TBG) are valuable aids in the diagnosis of thyroid disorders.

## 2. *BRIEF DESCRIPTION OF THE PRIOR ART*

Specific binding assay methods have undergone a technological evolution from the original competitive binding radioimmunoassay (RIA) in which a radioisotope -labeled antigen is made to compete with antigen from a test sample for binding to specific antibody. In the RIA technique, sample antigen is quantitated by measuring the proportion of radioactivity which becomes associated with the antibody by binding of the radiolabeled antigen (the bound-species of the labeled antigen) to the radioactivity that remains unassociated from antibody (the free-species) and then comparing that proportion to a standard curve. A comprehensive review of the RIA technique is provided by Skelly *et al*, *Clin. Chem.* *19*:146(1973). While by definition RIA

is based on the binding of specific antibody with an antigen or hapten, radiolabeled binding assays have been developed based on other specific binding interactions, such as between hormones and their binding proteins.

From radiolabeled binding assays have evolved nonradioisotopic binding assays employing labeling substances such as enzymes as described in U.S. Patents Nos. 3,654,090 and 3,817,837. Recently, further improved nonradioisotopic binding assays have been developed as described in U.S. Patents Nos. 4,134,792; 4,230,797; and 4,238,565, assigned to the present assignee, employing such unique labeling substances as enzyme modulators, enzyme substrates, coenzymes, chemiluminescent molecules, and enzyme prosthetic groups.

In particular, U.S. Patent No. 4,134,792 describes a specific binding assay employing an enzyme modulator, particularly an enzyme inhibitor, as the labeling substance. Where the label is an enzyme inhibitor, it is monitored by its ability to inhibit a predetermined enzymatic reaction. The use of methotrexate (4-amino-10-methylfolic acid), an inhibitor for the enzyme dihydrofolate reductase, as an enzyme inhibitor label is taught and a general structural formula and a preparative synthetic sequence is provided for methotrexate-labeled conjugates.

Additionally, Belgian Patent No. 864,856 describes enzyme modulator (e.g., enzyme inhibitor)-labeled specific binding assays and specifically exemplifies the preparation and use of methotrexate-labeled conjugates for the ligands digoxin, albumin and morphine.

The preparation of nonradioisotopically labeled iodothyronine conjugates in general is notoriously and uniquely fraught with difficulties and complexities. This is due to several factors, the principal of which are the hydrophobicity and low water solubility of the iodothyronines and the propensity of iodothyronines to undergo free radical decomposition and photo-deiodination.

Other literature of general interest to the preparation of conjugates comprising iodothyronines, particularly thyroxine, are U.S. Patent No. 4,040,907 describing enzyme-labeled iodothyronines and U.S. Patent No. 4,171,432 describing flavin adenine dinucleotide (FAD)-labeled iodothyronines. The preparation of unsymmetrical anhydrides of glutamic acid is described in *Org. Prep. Proc. Int. 11(2)*:67(1978). The preparation of methotrexate-fluorescein conjugates by this route is described in *J. Med. Chem. 18(5)*:526(1975).

## SUMMARY OF THE INVENTION

Labeled iodothyronine conjugates comprising the enzyme inhibitor methotrexate as the label are now provided having the formula:

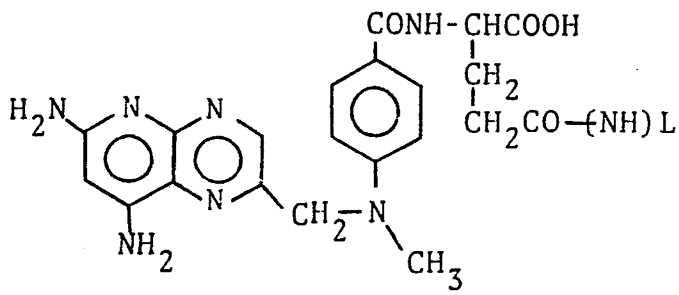

wherein -(NH)L is an iodothyronine, e.g., thyroxine or triiodothyronine, or a derivative thereof, coupled through an amide bond. The methotrexate-labeled conjugates are useful as labeled conjugates in specific binding assays such as immunoassays for determining iodothyronines in liquid samples or for determining iodothyronine uptake (e.g., T-3 uptake) of liquid samples. Either homogeneous or heterogeneous assay formats can be used, although a homogeneous format is generally preferred.

In one type of homogeneous immunoassay for determining an iodothyronine in a sample, the methotrexate-labeled conjugate (comprising the iodothyronine to be determined or a binding analog thereof) and any of the iodothyronine that is present in the sample are placed in competition for binding to a binding protein for the iodothyronine, e.g., an antibody raised against the iodothyronine. Two forms of the labeled conjugate result; a bound-form in which the labeled conjugate is bound by antibody and a free-form in which the conjugate is not so bound, with the proportion of the labeled conjugate between the two forms being a function of the

concentration of iodothyronine in the test sample. The ability of the conjugated methotrexate label to inhibit the enzyme dihydrofolate reductase is significantly less in the bound-form compared to the free-form. Accordingly, the proportion of labeled conjugate between the two forms can be assessed without having to physically separate the two forms, i.e., the assay is homogeneous, by adding the enzyme and measuring the amount of uninhibited enzyme activity resulting in the reaction mixture. Dihydrofolate reductase activity is preferably measured with an indicator comprising the substrate dihydrofolate and NADPH (nicotinamide adenine dinucleotide phosphate), and optionally a chromogenic substance responsive to the formation of tetrahydrofolate. Thus, reagent means are provided for determining an iodothyronine in a liquid sample, comprising (a) a methotrexate-labeled conjugate of said iodothyronine (b) a binding protein, e.g., antibody, for the iodothyronine, (c) dihydrofolate reductase, and (d) an indicator responsive to dihydrofolate reductase activity.

Iodothyronine uptake of a liquid sample is determined, in one example, by combining the sample with a methotrexate-labeled iodothyronine conjugate. Where the iodothyronine in the conjugate is triiodothyronine, the assay is for T-3 uptake. Binding proteins in the sample, predominantly thyroid binding globulin (TBG), bind the labeled conjugate, thereby significantly reducing the ability of the methotrexate label to inhibit dihydrofolate reductase. Upon addition of the enzyme, the amount of resulting uninhibited enzyme activity is a function of the amount of labeled conjugate taken up by binding to sample proteins. Thus, reagent means are provided for determining iodothyronine uptake of a liquid sample comprising (a) a methotrexate-labeled iodothyronine conjugate, (b) dihydrofolate reductase, and (c) an indicator responsive to dihydrofolate reductase activity.

## BRIEF DESCRIPTION OF THE DRAWINGS

Figs. 1, 1a, and 2 are flow diagrams of the synthetic routes illustrated in the Examples for preparing methotrexate-labeled iodothyronine conjugates.

DESCRIPTION OF THE PREFERRED EMBODIMENTS

The methotrexate-labeled iodothyronine conjugates of the present invention are prepared by peptide condensation between the δ-carboxyl group in methotrexate and an appropriate amino-iodothyronine compound. The iodothyronines themselves contain an amino function and therefore can be coupled to methotrexate directly. Alternatively, an amino-derivative of the iodothyronine can be prepared and used to couple to methotrexate. Usually, such an amino-iodothyronine derivative will be a compound of formula (*I*)

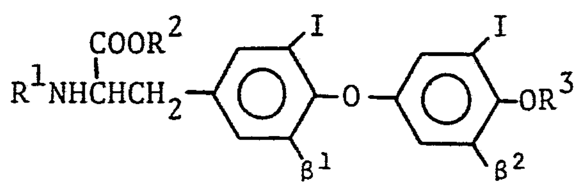

(*I*)

wherein $\beta^1$ and $\beta^2$ are, independently, hydrogen or iodine, and wherein one of $R^1$, $R^2$ and $R^3$ is $H_2NR$- and the other two are hydrogen, where R is a linking or spacer group.

Linking group R can vary widely and will be a bond or a chain, normally an aliphatic chain, comprising between 1 and 20 atoms, more usually between 1 and 10 atoms, excluding hydrogen, of which between 0 and 5 are heteroatoms selected from nitrogen, oxygen, and sulfur. Exemplary of useful linking groups R are linear and branched alkylenes comprising from 1 to as many as 12, more usually 8 or less, and normally less than 6, carbon atoms (e.g., methylene, ethylene, *n*-propylene, *iso*-propylene, *n*-butylene, and so forth). In addition, such alkylenes can contain substituent groups, in addition to the terminal amino substituent for coupling

to methotrexate, such as hydroxyl, halogen, carbonyl groups, carboxyl (including substituted carboxyls such as esters, amides, and substituted amides), cyano, and thiol. Linking group R may also contain or consist of substituted or unsubstituted aryl, aralkyl, or heteroalkyl groups (e.g., phenylene, phenethylene, and so forth). Additionally, such linkages may contain one or more heteroatoms selected from nitrogen, oxygen and sulfur in the form of ether, ester, amido, thio ether, amidino, sulfone or sulfoxide. Also, such linkages may include unsaturated groupings such as olefinic or acetylinic bonds, imino, or oximino groups. A preferred linking group is linear alkylene, i.e., $-(CH_2)_n-$ with $n$ being an integer from 2 through 12. Accordingly, the choice of linking group R is not critical to the present invention and may be selected by one of ordinary skill taking normal precautions to assure that stable compounds are produced.

The preparation of several different classes of useful amino-functionalized iodothyronines (*I*) will now be specifically exemplified.

A preferred class of amino-functionalized iodothyronines are the N-aminoalkyl iodothyronines of the formula:

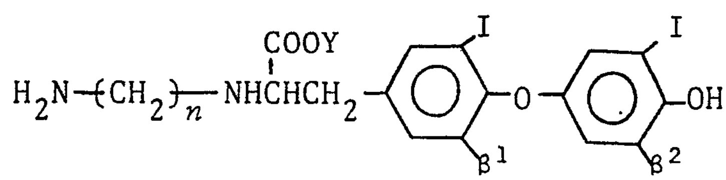

wherein $\beta^1$ and $\beta^2$ are defined as above, $n$ is an integer from 2 through 12, and Y is hydrogen or a carboxyl protecting group such as linear or branched alkyl, preferably lower alkyl ($C_{1-4}$) such as methyl, ethyl, $n$-propyl,

*iso*-propyl, *n*-butyl, etc. These N-derivatives can be prepared by first reacting ω-aminoalkylaldehyde diethyl acetal (*II*) with ethyl trifluoroacetate and triethylamine in ethanol to yield the N-trifluoroacetate (*III*).

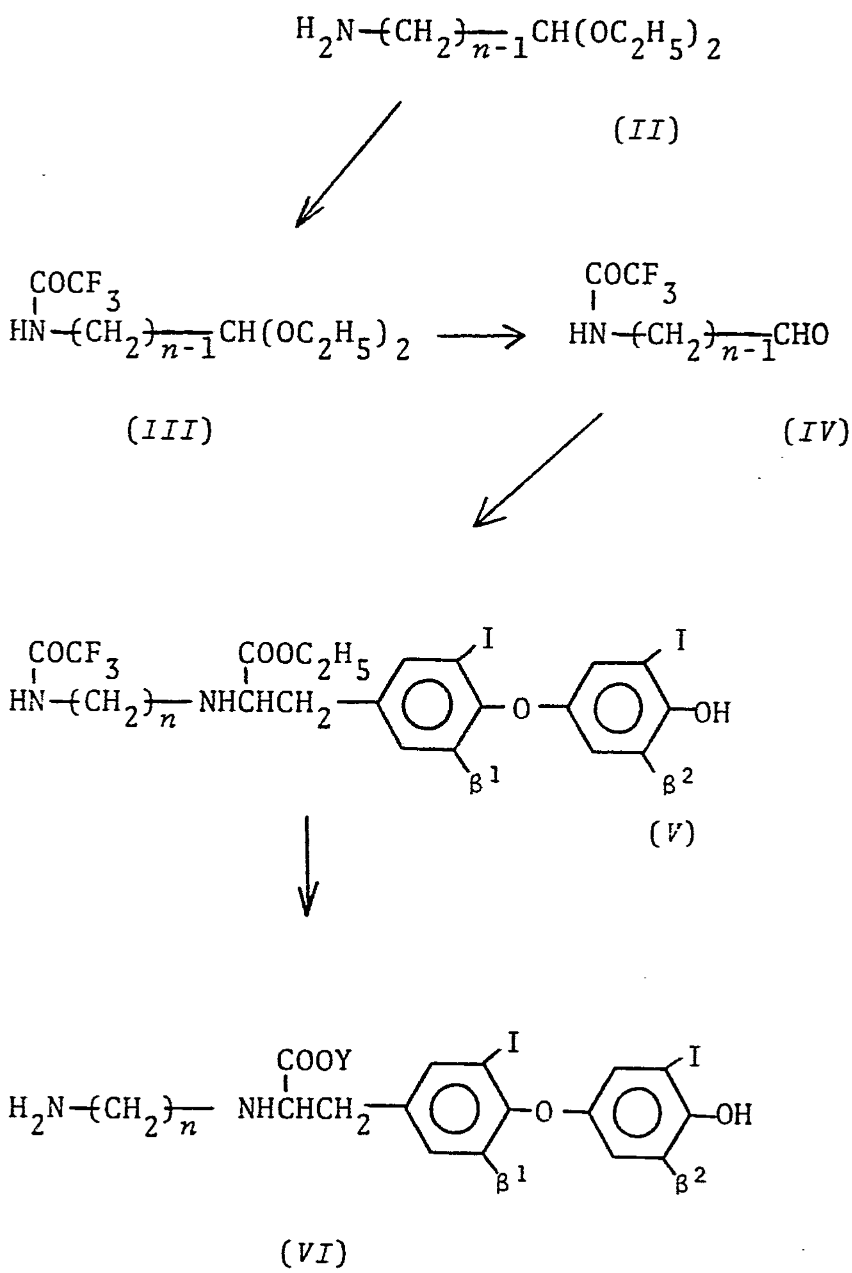

(*II*)

(*III*)

(*IV*)

(*V*)

(*VI*)

Hydrolysis yields the alkyraldehyde N-trifluoroacetate (*IV*) which upon reaction with iodothyronine ethyl ester hydrochloride and sodium cyanoborohydride in ethanol gives the N-(ω-aminoalkyl)-iodothyronine esters (*V*), Y=ethyl, which yield the N-(ω-aminoalkyl)-iodothyronines (*VI*), Y=hydrogen, upon saponification. Further details and exemplification of the preparation of N-aminoalkyl iodothyronine derivatives are given in the U.S. Patent Application filed on even date herewith, assigned to the present assignee, entitled "N-Aminoalkyl Iodothyronine Derivatives, Immunogens, and Antibodies. (Docket No. MS-1211)

Derivatization at the phenolic oxygen in the iodothyronines is exemplified by the preparation of O-carboxyalkyl derivatives by alkylation of the corresponding N-acetates as described in German OLS 2,737,802.

Functionalization at the carboxyl group in the iodothyronines is obtained by condensation of the iodothyronine with a diaminoalkane to form an N-(aminoalkyl)amide derivative, which can then be condensed with methotrexate anhydride by conventional amide-bond forming reactions.

The labeled conjugates of the present invention are useful in specific binding assays for determining iodothyronines or iodothyronine uptake in a liquid test sample, usually a biological fluid such as serum or plasma, where methotrexate is used as the label. The methotrexate label is preferably monitored by its ability to inhibit the enzyme dihydrofolate reductase. Accordingly, iodothyronines can be determined by combining the liquid test sample with a methotrexate-labeled iodothyronine conjugate of the present invention, a binding protein, e.g., antibody or fragment thereof, for the iodothyronine under assay, dihydrofolate reductase, and an indicator responsive to dihydrofolate reductase activity. The labeled conjugate, which is active as an inhibitor of dihydrofolate reductase, competes with iodothyronine in the sample for binding to the binding protein. Conjugate which becomes bound to the binding protein becomes less active as an inhibitor, and thus the level of resulting dihydrofolate reductase activity in the reaction mixture is inversely related to the amount of iodothyronine in the sample.

Similarly, the iodothyronine uptake, e.g., T-3 uptake, of a sample can be determined by combining the sample with labeled conjugate of the present invention, dihydrofolate reductase, and an indicator responsive to dihydrofolate reductase activity. Labeled conjugate which is taken up by binding proteins in the sample becomes less active as an enzyme inhibitor. Thus, the amount of resulting dihydrofoloate reductase activity in the reaction mixture is a direct function of the iodothyronine uptake value for the sample.

Any known indicator of dihydrofolate reductase activity can be used to monitor the enzyme activity in the reaction mixture. Usually, such indicator comprises a composition of reagents including NADPH and dihydrofolate. Enzymatic catalysis of the conversion of dihydrofolate to tetrahydrofolate in the presence of the cofactor NADPH results in the formation of oxidized cofactor NADP and these reactions are monitored by the decrease in absorbance at 340 nm. One can also measure NADP colorimetrically by including a chromogen which changes color on reduction by tetrahydrofolate. Such indicator chromogens are well known in the art and include tetrazolium dyes and ferric complexes of 2,2'-bipyridine or 1,10-phenanthroline.

Further details concerning specific binding assays employing enzyme inhibitor labels such as methrotrexate may be found in U.S. Pat. No. 4,134,792, assigned to the present assignee, and incorporated herein by reference.

The reagent means of the present invention comprises all of the essential chemical elements required to conduct a desired assay method encompassed by the present invention. The reagent means is presented in a commercially packaged form, as a composition or admixture where the compatability of the reagents will allow, in a test device configuration, or as a test kit, i.e., a packaged combination of one or more containers holding the necessary reagents. Accordingly, reagent means for the determination of an iodothyronine comprises a methotrexate-labeled conjugate of the present invention, a binding protein, e.g., antibody or fragment thereof, for the iodothyronine under assay, dihydrofolate reductase, and the indicator as described previously. Likewise, for the determination of iodothyronine

uptake, the reagent means comprises the labeled conjugate, dihydrofolate reductase, and the indicator. Of course, the reagent means can include other materials as are known in the art and which may be desirable from a commercial and user standpoint, such as buffers, diluents, standards, and so forth. Also preferred is a test device comprising reagent means of the present invention and a solid carrier member incorporated with the reagent composition. The various forms of such test device are described in U.S. Patent Application Serial No. 202,378, filed October 30, 1980, which is incorporated herein by reference.

The present invention will now be illustrated, but is not intended to be limited, by the following examples.

## EXAMPLE 1

### A. Preparation of Labeled Conjugate - Methotrexate-Aminobutylthyroxine Conjugate

The reaction sequence for the preparation of this labeled conjugate is shown in Diagram A in Figures 1 and 1a of the drawings.

#### 4-N-(Trifluoroacetyl)aminobutyraldehyde Diethylacetal (*2*), *n*=4.

To a mixture of 17.74 grams (g) of 90% 4-aminobutyraldehyde diethylacetal (*1*) [0.1 moles (mol)] and 22.1 milliliters (ml) triethylamine (0.16 mol) in 100 ml dry ethanol at 0°C under argon gas was added dropwise 21.3 g ethyl trifluoroacetate (0.16 mol) over 15 minutes. The mixture was allowed

to warm to room temperature overnight. The reaction volume was concentrated *in vacuo* (bath temperature <40°C), dissolved in ether, and washed with water and brine. Drying ($Na_2SO_4$), filtration, and evaporation of solvent *in vacuo* gave 25.3 g of brown oil. Fractional distillation 104-105°C (0.01 mm) provided the product (*2*), *n*=4, as a colorless oil (96%, 24.6 g).

Analysis: Calculated for $C_{10}H_{18}NF_3O_3$(MW 257.26: C, 46.7; H, 6.8; N, 5.5
Found: C,46.3; H, 7.0; N, 5.3.
PMR (60 mHz, $CDCl_3$): δ1.2 (t,J=7Hz,6H); 4.3 (m,4H); 3.2-4.0 (m,6H); 4.52 (t,J=5Hz,1H); 7.3 (bs,1H).
IR(neat): 1705 $cm^{-1}$

N-[4-N-(Trifluoroacetyl)aminobutyl]thyroxine Ethyl Ester (*4*), *n*=4, $\beta^1$=$\beta^2$=I.

4-N-(Trifluoroacetyl)aminobutyraldehyde diethylacetal (*2*), *n*=4, [3.097 g, 12 millimoles (mmol)] was stirred in a 36 ml mixture of tetrahydrofuran/acetic acid/water (1:1:1) for 48 hours under argon gas. The mixture was evaporated *in vacuo* to an oil which was added to 10.09 g (12 mmol) thyroxine ethyl ester hydrochloride (*3*), $\beta^1$=$\beta^2$=I, in 275 ml absolute ethanol under argon gas. Sodium cyanoborohydride [422 milligrams (mg), 6.8 mmol] was added to the mixture, which was allowed to stir 24 hours at room temperature. The mixture was filtered and concentrated *in vacuo* to a foam, which was dissolved in 40 ml 20% ethyl acetate-dichloromethane, filtered, and allowed to stand overnight. White crystals of product appeared which were collected, washed with dichloromethane, and dried to yield 4.32 g (37%), mp 202-204°C.

Analysis: Calculated for $C_{23}H_{22}N_2F_3I_4O_5 \cdot 2H_2O$:
C, 27.40; H, 2.70; N, 2.78
Found: C, 27.36; H, 2.45; N, 2.77
PMR (60 mHz, DMSO-$d_6$): δ1.13(t,J=3Hz, 3H); 1.67 (m,4H); 2.7-3.9 (m,6H); 4.2 (m + superimposed q, J=7Hz,3H); 7.12 (s,2H), 7.92 (s,2H); 9.65 (m,3H).
IR (KCl): 1720, 1745 $cm^{-1}$.

The mother liquor was concentrated *in vacuo*, dissolved in 10 ml ethyl acetate, and chromatographed on a Waters Prep 500® HPLC (Waters Associates, Inc., Milford, MA) using one Prep-Pak-500® 5.7 ID x 30 cm silica cartridge (Waters Associates, Inc., Milford, MA) as a stationary phase and 20% ethyl acetate-dichloromethane as the mobile phase.

First eluted from the column was an oil which was precipitated as a white powder (1.1 g) from dichloromethane-hexane, mp 64-68°C decomposed. It was identified as N,N-*bis*-[4-N-(trifluoroacetyl)aminobutyl] thyroxine ethyl ester on the basis of the following analytical data (7% yield).

Analysis: Calculated for $C_{29}H_{31}N_3F_6I_4O_6 \cdot H_2O$ (MW 1157.24):
C, 30.10; H, 2.87; N, 3.63
Found: C, 30.01; H, 2.45; N, 4.13
PMR (60 mHz, DMSO-$d_6$): δ1.15 (m + bt, J=7Hz,7H); 1.65 (m,4H); 2.9 (m,3H); 3.2 (m,4H); 3.7 (m,3H); 4.1 (m+q, J=7Hz, 3H); 5.73 (m,2H); 7.13 (s,2H); 7.88 (s,2H); 9.4(m,1H).
IR (KCl): 1720 $cm^{-1}$.

Next obtained from the column was an oil, which was precipitated as 3.886 g of a white powder from dichloromethane-hexane. Spectral data showed it to be identical with the initially formed crystals. The total yield of product (*4*), *n*=4, $\beta^1=\beta^2=I$, was 8.23 g (71%), mp 105-106°.

Analysis: Calculated for $C_{23}H_{23}N_2O_5I_4F_3$(MW 972.09):
C, 28.41; H, 2.39; N, 2.88
Found: C, 28.33; H, 2.40; N, 2.78

N-(4-Aminobutyl)thyroxine Ethyl Ester Bishydrochloride (*5*), *n*=4, $\beta^1=\beta^2=I$.

N-[4-N-(Trifluroacetyl)aminobutyl]thyroxine ethyl ester (*4*), *n*=4, $\beta^1=\beta^2=I$, (2.5 g, 2.6 mmol) was dissolved in 100 ml anhydrous ethanol. The solution was heated to reflux while treated with gaseous hydrochloric acid for 7 hours. The mixture was concentrated *in vacuo* and the residue twice precipitated from ethanol-ether to give 2.115 g of an off-white powder (79% yield) which analyzed as the bishydrochloride diethanolate (*5*), *n*=4, $\beta^1=\beta^2=I$.

Analysis: Calculated for $C_{21}H_{24}N_2I_4O_4\cdot 2HCl\cdot 2(C_2H_2O)$ (MW 1041.13):
C, 28.84; H, 3.68; N, 2.69
Found: C, 29.32; H, 3.28; N, 2.91
PMR (90 mHz, DMSO-$d_6$): δ1.13 (t, J=7Hz); 1.74 (m); 3.29 (m); 3.66 (m); 4.57 (m); 7.07 (s); 7.88 (s);8.70 (m); 10.0 (m); 10.6 (m).
IR (KCl): 1735, 2970 $cm^{-1}$.

Methotrexate-aminobutylthyroxine Conjugate (*6*), $n$=4, $\beta^1=\beta^2$=I.

To 232 mg methotrexate (0.51 mmol) and 70 microliters (μl) triethylamine (0.51 mmol) in 8 ml dimethylformamide under argon gas and subdued light at -10°C was added 68.4 μl isobutyl chloroformate (0.53 mmol). The mixture was stirred 1 hour at -10°C and 2 hours at room temperature. The reaction mixture was filtered into a second flask containing 474 mg N-(4-aminobutyl)thyroxine ethyl ester bishydrochloride bisethanolate (*5*), $n$=4, $\beta^1=\beta^2$=I, (0.05 mmol) and 210 μl triethylamine (1.52 mmol) in 5 ml dimethylformamide. The mixture was allowed to stir overnight in subdued light under argon gas. The mixture was preadsorbed onto a small amount of Silicar CC-7 (silicic acid; Mallindrodt, Paris, KY) and applied to the top of a 2.5 x 60 cm column of Silicar CC-7 packed with a 600:200:1 mixture of dichloromethane, methanol, and triethylamine. Elution of the column with this mixture gave 0.5 g of crude product which was precipitated from methanol and a 1:1 (v/v) mixture of ether and petroleum ether. Drying of the sample at 55°C (0.1 mm) gave 418 mg of an amorphous orange-red powder (64% yield), mp 152°, decomposed.

Analysis: Calculated for $C_{41}H_{44}N_{10}I_4O_8$(MW 1312.51):
C, 37.52; H. 3.38; N. 10.67
Found: C, 37.73; H, 3.62; N, 10.32
PMR (60 mHz, DMSO-$d_6$): δ 1.12 (t,J=7Hz, 3H); 1.38 (m,6H); 2.13 (m,4H); 2.5-3.6 (m,7H, containing s, 3.23 δ); 4.07 (q,J=7Hz,2H); 4.12 (m,1H); 5.92 (m,3H); 6.67 (m,1H); 6.88 (m,1H); 6.99 (m,1H); 7.13 (s,2H); 7.77 (m,6H); 7.88 (s,2H); 8.58 (s,1H).
IR(KCl): 3410, 1720, 1600-1670, 1530-1560, 1500, 1430-1450 $cm^{-1}$.

The previously obtained powder (348 mg, 0.27 mmol) was dissolved in 4 ml methanol under argon gas and subdued light and was treated with 2 ml 1 N sodium hydroxide. The sample was stirred at 30°C for 2 hours. The product was precipitated by addition of acetic acid, filtered, washed with water, and dried overnight (55°C, 0.1 mm). An amorphous yellow powder was obtained (249 mg, 69%). Thin layer chromatography (silicon dioxide eluted with the lower phase of a 1:1:1 chloroform-methanol-ammonium hydroxide mixture) revealed the product (*6*), $n$=4, $\beta^1=\beta^2=I$, ($R_f$=0.42) accompanied by approximately 5% of the coupling product of aminobutylthyroxine with the α-glutamoyl carboxyl ($R_f$ 0.46) of methotrexate.

Analysis: Calculated for $C_{39}H_{40}N_{10}I_4O_8 \cdot 3H_2O$ (MW 1338.51):
C, 35.00; H, 3.46; N, 10.46
Found: C, 35.34; H, 3.57; N, 9.90
PMR (90 mHz, DMSO-$d_6$): δ1.43 (m,6H); 2.09 (m,4H); 2.69 (m,2H); 2.95 (m,3H); 3.15 (s,3H); 3.40 (m,1H); 4.40 (m,3H); 4.77 (bs,3H); 5.47 (m,1H); 6.57 (bs,2H); 6.77 (s,1H); 6.87 (s,1H); 7.7 (m,6H); 7.83 (s,2H); 8.38 (m,1H); 8.56 (s,1H).
IR(KCl): 3400, 1625, 1600 $cm^{-1}$.

* * * * * *

The above-described synthesis of the methotrexate-aminobutylthyroxine conjugate (*6*), $n$=4, $\beta^1=\beta^2$=I, can be modified to yield derivatives wherein $n$=2-12 and wherein an iodothyronine other than thyroxine is incorporated into the conjugate as follows. To prepare conjugates wherein $n$=2-12, the starting material 4-aminobutyraldehyde diethylacetal is replaced with the appropriate ω-aminoalkanal. To prepare conjugates wherein another iodothyronine is incorporated into the conjugate, the starting material thyroxine ethyl ester (*3*), $\beta^1=\beta^2$=I, is replaced with the appropriate iodothyronine ethyl ester.

B. Dihydrofolate Reductase Assay

The standard assay consisted of 0.10 mM dihydrofolate (DHF), 0.10 mM NADPH, 0.001 unit/bovine liver dihydrofolate reductase (Sigma Chemical Co., St. Louis, MO) and 0.1 M sodium phosphate (pH 6.0) in a final volume of 2.0 ml. The reaction mixture without dihydrofolate was incubated at 37°C for 5 minutes, then the reaction was initiated by adding dihydrofolate, and an initial absorbance at 340 nm was recorded with a Gilford 250 spectrophotometer (Gilford Instrument Laboratories, Oberlin, OH). The reaction was allowed to proceed for 20 minutes at 37°C and then a final reading was recorded. The enzyme activity was expressed as the change in absorbance (ΔA) at 340 nanometers (nm) that occurred during the 20 minute (min) reaction.

C. Inhibition of Dihydrofolate Reductase by the Labeled Conjugate

Various concentrations of methotrexate-aminobutylthroxine conjugate were incubated with the standard concentrations of NADPH, dihydrofolate reductase and buffer for 5 minutes at 37°C. The reaction was initiated as discussed above with the addition of dihydrofolate. The results presented in Table 1 below show that the methotrexate-aminobutylthyroxine was an effective inhibitor of dihydrofolate reductase.

TABLE 1

| 0.1M Sodium Phosphate Buffer pH 6.5 (µl)) | Methotrexate-Aminobutyl-Thyroxine 0.16 µM (µl) | NADPH 2.1 mM (µl) | Dihydrofolate Reductase 0.04 U/ml (µl) | Dihydrofolate 3.5 mM (µl) | $\Delta A_{340}$/20 Min |
|---|---|---|---|---|---|
| 1800 | - | 95 | 50 | 55 | 0.645 |
| 1775 | 25 | 95 | 50 | 55 | 0.566 |
| 1750 | 50 | 95 | 50 | 55 | 0.495 |
| 1700 | 100 | 95 | 50 | 55 | 0.433 |
| 1600 | 200 | 95 | 50 | 55 | 0.306 |

D. Titration of Antibody to Thyroxine with the Labeled Conjugate

Various concentrations of antibody to thyroxine and the standard concentrations of NADPH, 6 nM methotrexate-aminobutylthyroxine and buffers were incubated for 2 minutes at room temperature. Dihydrofolate reductase was then added, and the incubation was continued for 5 minutes at 37°C. The enzyme catalyzed reaction was initiated by addition of dihydrofolate and monitored as described above. The results in Table 2 below show that the enzyme activity increased as the antibody level increased.

TABLE 2

| 0.1M Sodium Phosphate Buffer pH 6.5 (µl) | Antibody to Thyroxine 1:9 dilution (µl) | Methotrexate-Aminobutyl-Thyroxine 0.16 µM (µl) | NADPH 1.9 mM (µl) | Dihydrofolate Reductase 0.04 U/ml (µl) | Dihydrofolate 3.3 mM (µl) | $\Delta A_{340}$/20 Min |
|---|---|---|---|---|---|---|
| 1785 | - | - | 105 | 50 | 60 | 0.691 |
| 1710 | - | 75 | 105 | 50 | 60 | 0.499 |
| 1700 | 10 | 75 | 105 | 50 | 60 | 0.579 |
| 1690 | 20 | 75 | 105 | 50 | 60 | 0.602 |
| 1670 | 40 | 75 | 105 | 50 | 60 | 0.664 |
| 1630 | 80 | 75 | 105 | 50 | 60 | 0.656 |
| 1550 | 160 | 75 | 105 | 50 | 60 | 0.667 |

E. Competitive Binding Assay for Thyroxine

The standard concentrations of NADPH, buffer, antibody to thyroxine and various concentrations of thyroxine were incubated at room remperature for a minimum of 30 seconds. Then 100 µl of 0.16 µM methotrexate-aminobutylthyroxine was added and the mixture was incubated at room temperature for 2 minutes. Following the second incubation, dihydrofolate reductase was added and the mixture was incubated an additional 5 minutes at 37°C. The reaction was initiated and monitored in the usual manner. The results in Table 3 below show that the enzyme activity decreased as the thyroxine level increased.

MS-1210

TABLE 3

| 0.1 Sodium Phosphate Buffer pH 6.5 (μl) | Thyroxine $1 \times 10^{-7}$M (μl) | Antibody to Thyroxine 1:4 Dil (μl) | Methotrexate-Aminobutyl-Thyroxine 0.16 μM (μl) | NADPH 2.1 mM (μl) | Dihydrofolate Reductase 0.04 U/ml (μl) | Dihydro-folate 3.5 mM (μl) | $\Delta A_{340}$/ 20 Min |
|---|---|---|---|---|---|---|---|
| 1800 | - | - | - | 95 | 50 | 55 | 0.620 |
| 1700 | - | - | 100 | 95 | 50 | 55 | 0.458 |
| 1670 | - | 30 | 100 | 95 | 50 | 55 | 0.645 |
| 1650 | 20 | 30 | 100 | 95 | 50 | 55 | 0.637 |
| 1630 | 40 | 30 | 100 | 95 | 50 | 55 | 0.610 |
| 1590 | 80 | 30 | 100 | 95 | 50 | 55 | 0.595 |
| 1550 | 120 | 30 | 100 | 95 | 50 | 55 | 0.595 |
| 1510 | 160 | 30 | 100 | 95 | 50 | 55 | 0.585 |
| 1470 | 200 | 30 | 100 | 95 | 50 | 55 | 0.584 |

Thus, an assay was provided for determining thyroxine in a liquid sample.

## EXAMPLE 2

### A. Preparation of Labeled Conjugate - Methotrexate-thyroxine Conjugate

The reaction sequence for the preparation of this labeled conjugate is shown in Diagram B in Figure 2 of the drawings.

A solution of 464 mg (1 mmol) of methotrexate in 15 ml of dry dimethylformamide (DMF) was cooled to -10°C while stirring under argon. To this was added 0.14 ml (1 mmol) of triethylamine and 136 mg (1 mmol) of isobutyl chloroformate. After stirring for 1 hour at this temperature, 841 mg (1 mmol) of the hydrochloride salt of thyroxine ethyl ester was added. Stirring was continued at -10°C for 2 hours, then at room temperature overnight. Ten grams of silica gel was added and the solvent removed under high vacuum. The impregnated adsorbent was placed atop a column of 100 g of silica gel made up in absolute ethanol. The column was eluted with 1 liter of ethanol and the eluate discarded. It was then eluted with a gradient of 2 liters of absolute ethanol to 2 liters of 9:1 (v/v) ethanol: 1M aqueous triethylammonium bicarbonate. Twenty ml fractions were collected.

Fractions 81 to 140 were combined and evaporated to give 500 mg of the methotrexate-thyroxine ethyl ester conjugate as a yellow powder which was not further characterized. It was added to a mixture of 5 ml water, 2 ml methanol, and 80 mg (5 equivalents) of sodium hydroxide. After stirring overnight at room temperature solvent was removed *in vacuo*, the residue taken

taken up in 10 ml water, and filtered through celite. The filtrate was acidified with 0.6 ml of glacial acetic acid. A yellow precipitate formed which was filtered, washed with water, and dried at 50°C for 2 hours under vacuum. When dry this amounted to 280 mg (23% yield) of the thyroxine-methotrexate conjugate *(7)*, $\beta^1=\beta^2=I$, as an amorphous yellow powder.

Analysis: Calculated for $C_{35}H_{30}N_9I_4O_8$: C, 34.68; H, 2.49; N, 10.40.
Found: C, 34.53; H, 2.76; N, 10.41.
UV Spectrum ($H_2O$, pH 5.5): $\lambda_{max}$=303 nm ($\varepsilon_{max}$=24,100).

*          *          *

The above-described synthesis of the methotrexate-thyroxine conjugate *(7)*, $\beta^1=\beta^2=I$, can be modified to prepare conjugates for other iodothyronines by replacing the starting material thyroxine ethyl ester with the appropriate iodothyronine ethyl ester.

B. Titration of Antibody to Thyroxine with the Labeled Conjugate

Thyroxine-free serum was prepared by treatment of pooled human serum with charcoal [Larsen *et al*, *J. Clin. Endocrinol. Metab.* *37*:177(1973)]. All reagents except thyroxine-free serum or as otherwise indicated below were prepared in 0.1 M sodium phosphate buffer, pH 6.5, containing 0.3 M potassium chloride and 0.005% sodium azide.

Reagent 1 - Mixture of 6.5 ml of 10 mM 8-anilinonaphthalene sulfonic acid and 10 ml of thyroxine-free serum.

Reagent 2 - Mixture of 1.0 ml of bovine liver dihydrofolate reductase (0.152 units/ml) and 1.0 ml of 11 mM thiazoyl blue.

Reagent 3 - 0.114 mM dihydrofolate in 0.085 M sodium phosphate buffer, pH 6.2, 0.255 mM potassium chloride, and 0.043% sodium azide.

Reagent 4 - Rabbit antibody to thyroxine diluted 10-fold in buffer.

Reagent 5 - 175 mM methotrexate-thyroxine conjugate (Part A above) in 10 mM sodium carbonate buffer, pH 9.5.

Reagent 6 - 10 mM NADPH in 10 mM sodium carbonate buffer, pH 9.5.

Various amounts of Reagent 4 (indicated in Table 4 below) were mixed with 40 µl of Reagent 5, 90 µl of Reagent 6, and sufficient buffer to make a final volume of 625 µl. Then 175 µl of Reagent 1 was added to each followed by 250 µl of buffer and the incubation was

continued. After 10 minutes from the addition of Reagent 3, the absorbance at 560 nm was recorded for each reaction mixture and shown in Table 4.

TABLE 4

| Antibody Solution - Reagent 4 (µl) | Average Absorbance (560 nm) |
|---|---|
| - | 0.478 |
| 20 | 0.538 |
| 40 | 0.581 |
| 80 | 0.632 |
| 160 | 0.689 |
| 320 | 0.737 |

C. Competitive Binding Assay for Thyroxine

A series of reaction mixtures were set up in cuvettes by the following additions:

1. 250 µl of buffer plus 100 µl of a serum standard containing a known concentration of thyroxine.
2. 250 µl of buffer plus 100 µl of 8.1 mM 8-anilinonaphthalene sulfonate containing 8.7 µl of thyroxine antibody.
3. 250 µl of buffer plus 100 µl of 9 mM NADPH in 10 mM sodium carbonate buffer, pH 9.5.
4. 250 µl of buffer plus 100 µl of 5.4 mM thiazoyl blue containing 0.075 units/ml of dihydrofolate reductase.
5. 250 µl of buffer plus 100 µl of 0.11 mM dihydrofolate.

After step 4, the reactions were incubated at 37°C for 10 minutes. Then after step 5, the incubation was continued for 20 minutes and the absorbance read at 560 nm. The results were as follows:

TABLE 5

| Thyroxine Concentration (μg/dl) | Absorbance (560 nm) |
|---|---|
| 0 | 0.603 |
| 1.0 | 0.585 |
| 2.5 | 0.595 |
| 5.0 | 0.582 |
| 15.2 | 0.545 |
| 30.3 | 0.527 |

Thus, an assay was provided for determining thyroxine in a liquid sample.

CLAIMS:

1. A methotrexate-labeled iodothyronine conjugate of the formula:

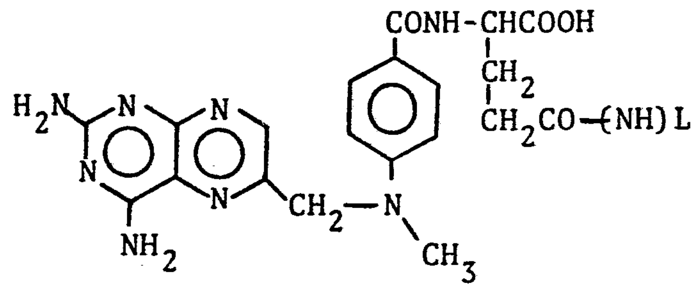

wherein -(NH)L is an iodothyronine, or a derivative thereof, coupled through an amide bond.

2. A conjugate of Claim 1 wherein said iodothyronine is thyroxine.

3. A conjugate of claim 1 or 2 wherein -(NH)L is of the formula:

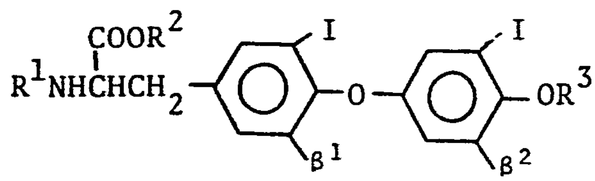

wherein $\beta^1$ and $\beta^2$ are, independently, hydrogen or iodine, and wherein either $R^1$ is a bond and $R^2$ and $R^3$ are hydrogen, or one of $R^1$, $R^2$ and $R^3$ is -NHR- and the other two are hydrogen, where R is a linking group.

4. A conjugate according to claims 1 to 3 of the formula:

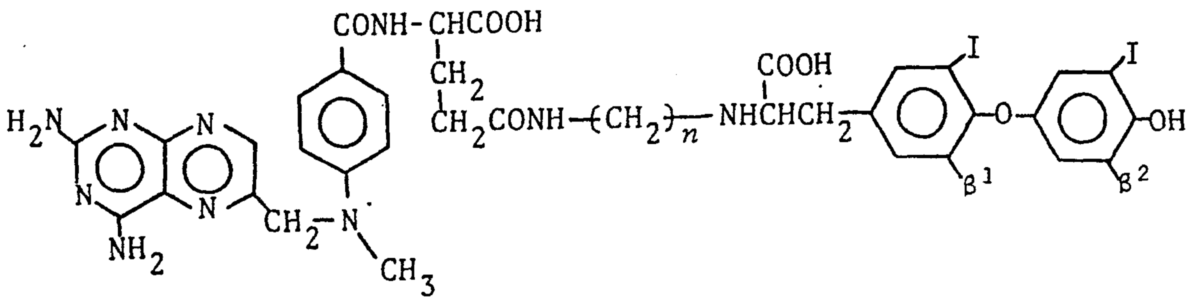

wherein $n$ is an integer from 2 through 12, and $\beta^1$ and $\beta^2$ are, independently, hydrogen or iodine.

5. A method for determining an iodothyronine in a liquid sample, comprising the steps of:

(a) combining said liquid sample and

(i) a methotrexate-labeled iodothyronine conjugate of Claims 1 to 4,

(ii) a binding protein for said iodothyronine,

(iii) dihydrofolate reductase, and

(iv) an indicator responsive to dihydrofolate reductase activity

to form a reaction mixture, and

(b) measuring the indicator response in said reaction mixture as a function of the iodothyronine in the sample.

6. A method for determining iodothyronine uptake of a liquid sample, comprising the steps of:

(a) combining said liquid sample and

(i) a methotrexate-labeled iodothyronine conjugate of Claims 1 to 4,

(ii) dihydrofolate reductase, and

(iii) an indicator responsive to dihydrofolate reductase activity

to form a reaction mixture, and

(b) measuring the indicator response in said reaction mixture as a function of iodothyronine uptake of said sample.

7. Reagent means for the determination of an iodothyronine in a liquid sample, comprising:

(a) a methotrexate-labeled iodothyronine conjugate of Claims 1 to 4,

(b) a binding protein for said iodothyronine,

(c) diydrofolate reductase, and

(d) an indicator responsive to dihydrofolate reductase activity.

8. Reagent means for the determination of iodothyronine uptake of a liquid sample, comprising:

(a) a methotrexate-labeled iodothyronine conjugate of Claims 1 to 4,

(b) dihydrofolate reductase, and

(c) an indicator responsive to dihydrofolate reductase activity.

9. A test device for the determination of an iodothyronine in a liquid sample, comprising the reagent means of Claim 7 and a solid carrier member incorporated therewith.

10. A test device for the determination of iodothyronine uptake of a liquid sample, comprising the reagent means of Claim 8 and a solid carrier member incorporated therewith.

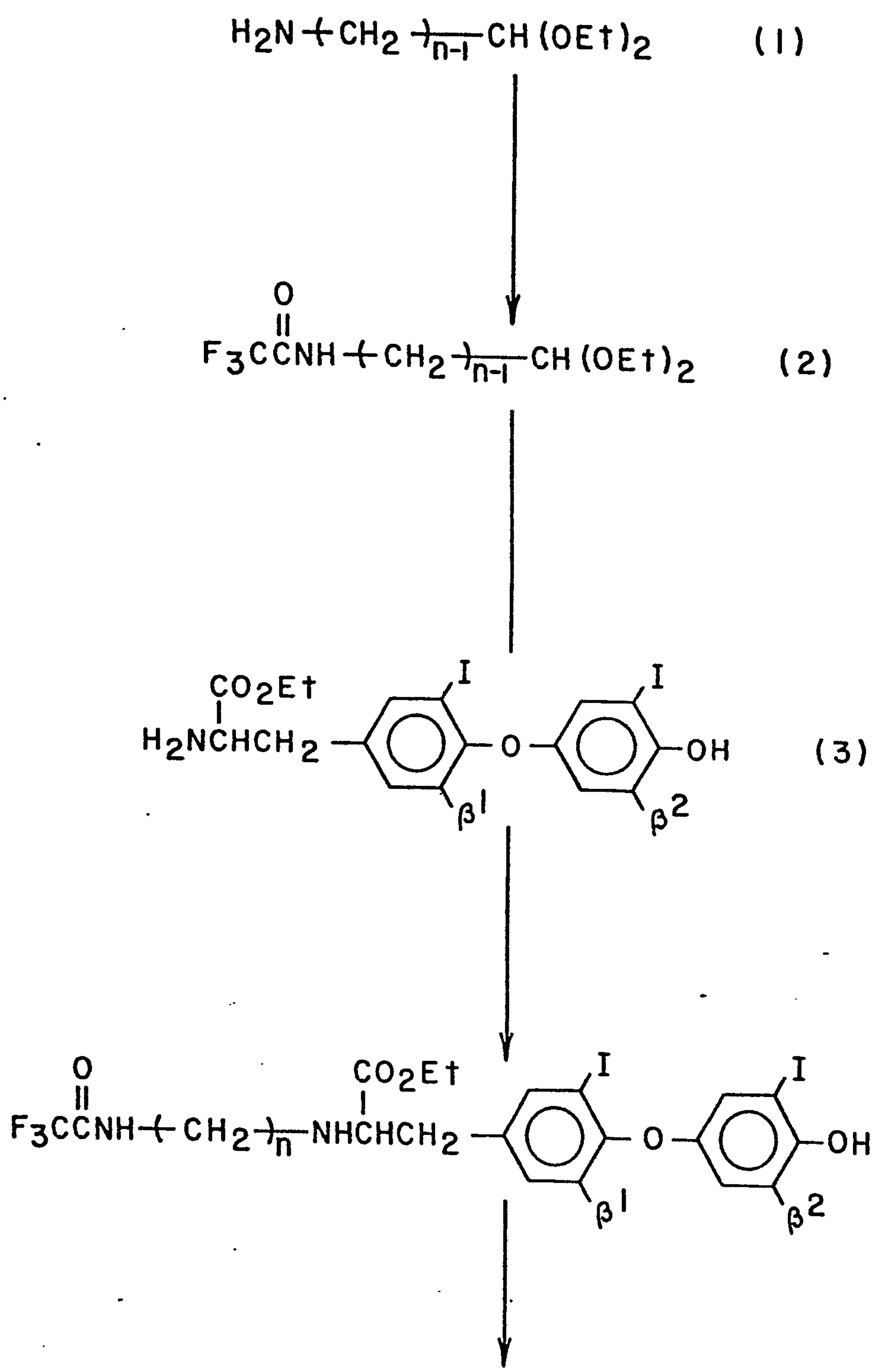

(1)

(2)

(3)

(4)

FIG. I

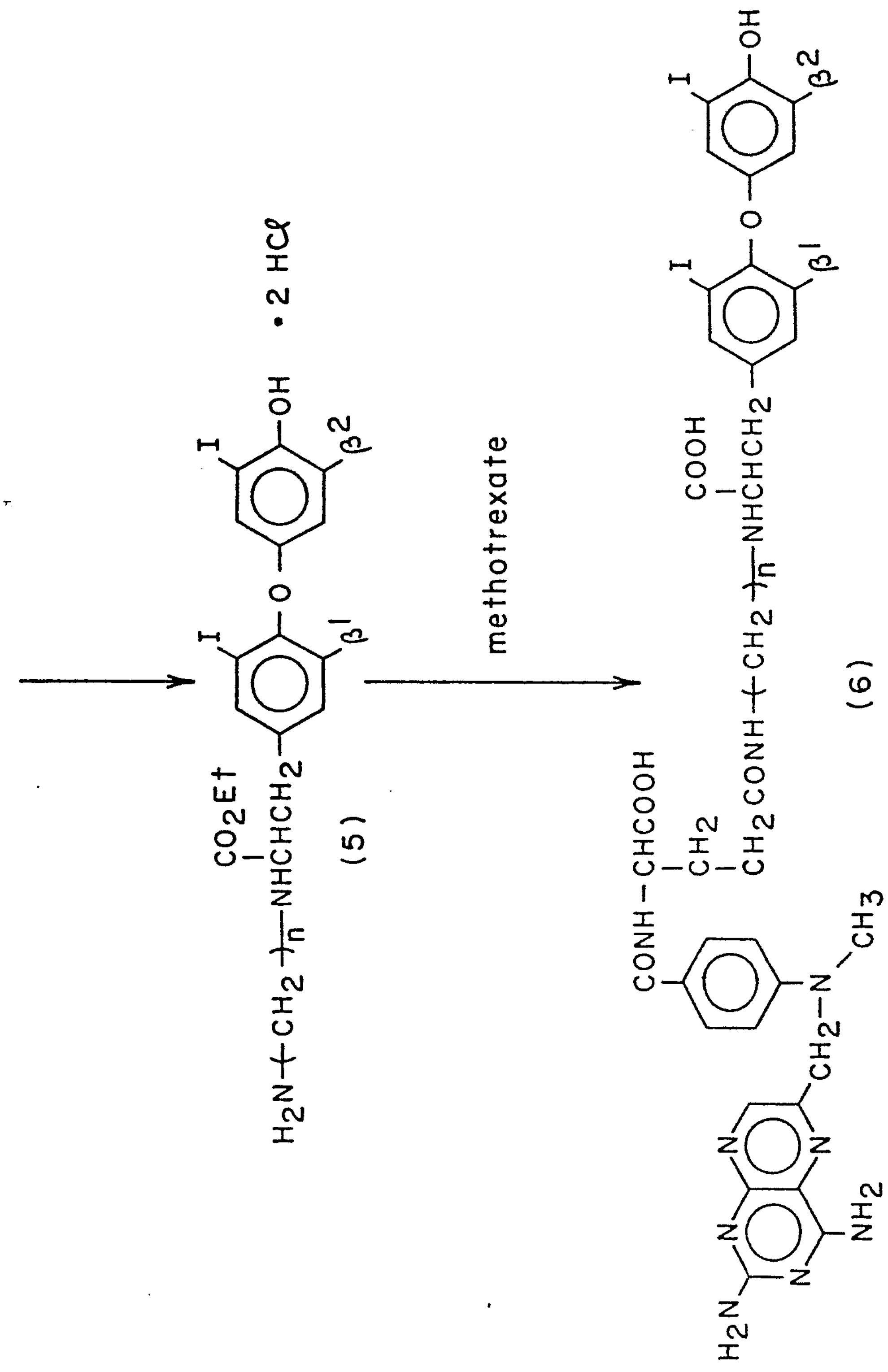


(5)

(6)

FIG. 1a

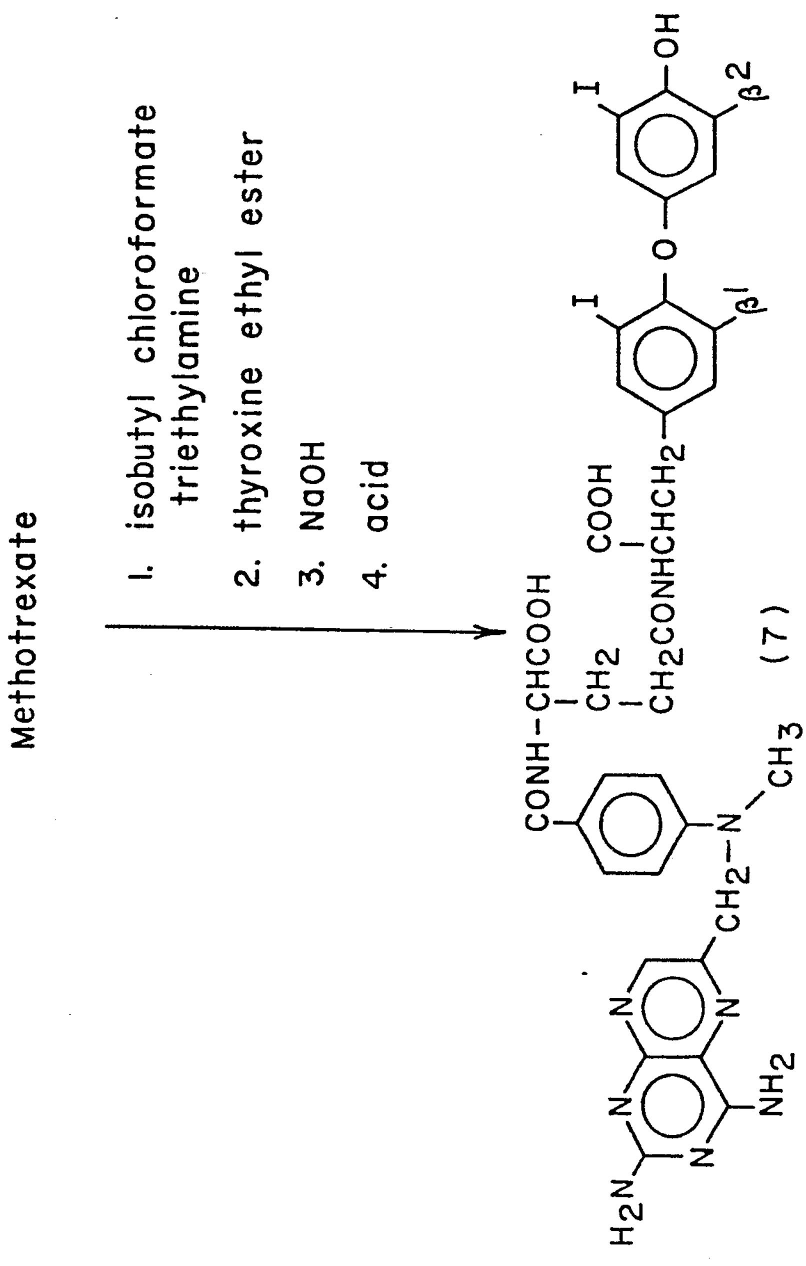
Methotrexate
1. isobutyl chloroformate
triethylamine
2. thyroxine ethyl ester
3. NaOH
4. acid

(7)

FIG. 2

European Patent Office

**EUROPEAN SEARCH REPORT**

0079489

Application number

EP 82 10 9831

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | G 01 N 33/78 |
| A,D | US-A-4 134 792 (R.C. BOGUSLASKI et al.) * Claim 1; column 6, lines 26-47; column 7, lines 1-24 | 1,5 | |
| | --- | | |
| A,D | US-A-4 238 565 (W.E. HORNBY et al.) | | |
| | --- | | |
| A,D | US-A-4 171 432 (R.J. CARRICO et al.) | | |
| | --- | | |
| A | DE-A-3 003 959 (ABBOTT LABORATORIES) | | |
| | ----- | | |
| | | | TECHNICAL FIELDS SEARCHED (Int. Cl. 3) |
| | | | G 01 N 33/54<br>G 01 N 33/78 |

The present search report has been drawn up for all claims

| Place of search | Date of completion of the search | Examiner |
|---|---|---|
| BERLIN | 24-01-1983 | SCHWARTZ K |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82